# EUROPÄISCHE PATENTSCHRIFT

(11) **EP 0 696 457 B1**
(45) Veröffentlichungstag und Bekanntmachung des Hinweises auf die Patenterteilung: **10.02.1999**
(21) Anmeldenummer: 94112152.7
(22) Anmeldetag: 03.08.1994
(51) Int. Cl.: A61L 9/03, A01M 1/20

(54) **Elektrische Vorrichtung zum Verdunsten von Wirkstoffen**
Electric vaporizer for active substances
Appareil électrique de vaporisation de substances actives

(43) Veröffentlichungstag der Anmeldung: 14.02.1996
(73) Patentinhaber: Steinel GmbH & Co. KG, D-33442 Herzebrock-Clarholz (DE)
(72) Erfinder: Steinel jr., Heinrich Wolfgang, D-86825 Bad Wörishofen (DE)
(74) Vertreter: Grünecker, Kinkeldey, Stockmair & Schwanhäusser Anwaltssozietät

(56) Entgegenhaltungen:
- EP-A- 0 290 159
- EP-A- 0 362 397
- WO-A-88/05310
- DE-U- 9 104 709

## Beschreibung

Die vorliegende Erfindung bezieht sich auf eine elektrische Vorrichtung zum Verdunsten von Wirkstoffen gemäß dem Oberbegriff des Patentanspruchs 1.

Solche Vorrichtungen sind zunehmend populär geworden, beispielsweise zum Verdampfen von Duft- und Aromastoffen, um Kleininsekten, wie beispielsweise Stechmücken etc. zu vertreiben. Diese Geräte werden bevorzugt mit einem im Gehäuse integrierten Anschlußstecker ausgeführt, so daß das Gerät unmittelbar in eine Wandsteckdose eingesteckt werden kann.

Es besteht ein Problem dahingehend, daß das Gerät normalerweise nur in einer bestimmten Position betrieben werden kann, da der Wirkstoff meist in einem flüssigen Trägermaterial oder einer Paste gebunden ist und deshalb nur in einer bestimmten Stellung des Geräts ein Auslaufen des Trägermaterials verhindert wird, die Buchsen einer Wandsteckdose aber auf einer horizontalen oder vertikalen Linie liegen können.

Zur Lösung dieses Problems gibt es bereits Geräte, die einen im Gehäuse angeordneten, um einen Winkel von etwa 90° drehbaren Anschlußstecker aufweisen. Dadurch ist es möglich, das Gehäuse des Geräts in die erforderliche Betriebsstellung zu schwenken.

Es sind Geräte bekannt, beispielsweise aus der EP-A-362 397 die mit einem elektrischen Schalter versehen sind, mit dem die Heizung an- oder abschaltbar ist. Das Gerät braucht dabei zum Abschalten nicht aus der Wandsteckdose genommen zu werden.

Der vorliegenden Erfindung liegt die Aufgabe zugrunde, eine Vorrichtung der eingangs genannten Art anzugeben, die mit einem Ein/Aus-Schalter und mit einer Anpassung an senkrechte oder horizontale Wandsteckdosen versehen ist, und dennoch einfach aufgebaut ist.

Diese Aufgabe wird ausgehend von einer gattungsbildenden Vorrichtung dadurch gelöst, daß der Anschlußstecker mit einem ersten Drehkupplungsteil versehen ist, welches mit einem im Gehäuse angeordneten zweiten Drehkupplungsteil in Eingriff steht und das in jedem der Drehkupplungsteile Kontakte angeordnet sind, die in und außer Kontakt mit den jeweils ihnen zugeordneten Kontakten des anderen Drehkupplungsteils bringbar sind.

Die Kontakte und die zugehörigen Gegenkontakte auf den beiden Drehkupplungsteilen sind derart angeordnet, daß sich in zwei um 90° versetzte Stellungen jeweils eine durchgehende elektrische Verbindung zwischen den Anschlußsteckerkontakten und der Heizung ergibt und dazwischen ein Winkelbereich mit einer Schaltstellung entsteht, in der die Verbindung geöffnet ist.

Durch die Ausbildung der Kontakte und der zugehörigen Gegenkontakte auf den beiden Drehkupplungsteilen ergibt sich ein vereinfachter Aufbau der Vorrichtung, die durch Verdrehen des Gehäuses gegenüber dem Anschlußstecker ein- und ausgeschaltet wird. Dadurch, daß die Vorrichtung zwei um 90° versetzte Einschaltstellungen besitzt, kann die Vorrichtung immer eine bestimmte Betriebsposition einnehmen, welche unabhängig davon ist, ob das Gerät in eine senkrechte oder horizontale Wandsteckdose eingesteckt ist.

Darüber hinaus ist für einen Bediener bereits aus der Stellung des Gehäuses leicht erkennbar, in welchem Betriebszustand sich das Gerät befindet, da in der ausgeschalteten Stellung das Gehäuse eine andere Position besitzt als in der eingeschalteten Stellung der Vorrichtung.

Vorzugsweise ist der Winkelbereich mit geschlossener Verbindung wesentlich kleiner als der Winkelbereich mit geöffneter Verbindung, insbesondere beträgt das Verhältnis des Winkelbereichs mit geschlossener Verbindung zum Winkelbereich mit geöffneter Verbindung 1:5.

In einer vorteilhaften Ausgestaltung weist die Heizung wenigstens einen PTC-Widerstand auf, der zwischen zwei metallischen Elektroden liegt, die über elektrische Verbindungsleitungen mit den Kontakten des gehäuseseitigen Drehkupplungsteils verbunden sind. PTC-Heizwiderstände sind dafür bekannt, daß sie sich in einer von ihrer Dimensionierung abhängigen Temperatur selbst stabilisieren.

Der Aufbau der Vorrichtung vereinfacht sich erheblich, wenn die Verbindungsleitungen, die Kontakte, der Wärmetauscher und ggf. die Elektroden einstückig aus einem Stanzblechteil gebildet sind, das nach dem Stanzen erforderlichenfalls abgebogen und an den Stellen, an denen eine erhöhte Formsteifigkeit bzw. eine Wärmeisolierung erforderlich ist, mit einem Kunststoff umspritzt ist. Die Ausbildung der wesentlichen elektrisch leitenden Teile auf einem einzigen Stanzblech vereinfacht die Vorrichtung erheblich, da die sonst üblichen Litzenverbindungen entfallen. Darüber hinaus erhalten die elektrisch leitenden Teile durch den Kunststoff eine erhöhte Festigkeit, wodurch die Haltbarkeit der Vorrichtung verbessert und die elektrische Verlustleistung vermindert ist.

In einer besonderen Ausgestaltung sind die elektrischen Verbindungsleiter in dem Bereich zwischen den Kontakten des gehäuseseitigen Drehkupplungsteils in Form zweier einander gegenüberliegender Viertelringe ausgebildet und die Verbindungsleiter an einer Stelle zwischen den Viertelringen und den Elektroden um einen Winkel von etwa 90° gegenüber den Elektroden abgeknickt. Vorzugsweise sind die Viertelringe mit Kunststoff derart umspritzt, so daß sich ein nahezu geschlossener Kreisring ergibt.

In diesem Ausführungsbeispiel steht das gehäuseseitige Drehkupplungsteil nach dem Abknicken senkrecht zu der Ebene, in der die Elektroden und der Wärmetauscher ausgebildet ist. Dies vereinfacht den Herstellungsprozeß erheblich, da alle Herstellungsschritte in einer Ebene ausgeführt werden können und danach nur ein Abknicken der Verbindungsleiter erforderlich ist, um das gehäuseseitige Drehkupplungsteil in die erforderliche senkrechte Stellung zu bringen, in der es in das Gehäuse eingesetzt wird.

Vorzugsweise ist das Drehkupplungsteil mit dem Anschlußstecker starr verbunden und in der Wand des Gehäuses vorzugsweise um einen Winkel von 90° drehbar geführt. Durch eine solche Ausbildung wird sichergestellt, daß der Bediener das Gerät nur den zwischen zwei um 90° gesetzten Einschaltstellungen verdrehen kann.

In einer vorteilhaften Weiterbildung sind die Drehkupplungsteile mittels einer Rastverbindung miteinander in Eingriff gehalten, wodurch definierte Schaltzustände der Vorrichtung gewährleistet werden. Beispielsweise berühren sich in den Einschaltstellungen die Kontakte des einen Drehkupplungsteils mit den Gegenkontakten des anderen Drehkupplungsteils und durch die Rastverbindung wird ein enges Anliegen der Kontakte unterstützt.

Die vorliegende Erfindung wird im folgenden anhand der Figuren näher beschrieben, welche zeigen:
- Fig. 1: eine Seitenansicht der Vorrichtung,
- Fig. 2: eine Seitenansicht der in dem Gehäuse gelagerten Heizung der Vorrichtung,
- Fig. 3: eine Sicht von oben auf die Heizung, die in Fig. 2 dargestellt ist,
- Fig. 4: eine Seitenansicht auf den Anschlußstecker und das erste Drehkupplungsteil der vorliegenden Erfindung,
- Fig. 5: eine Ansicht von vorne auf das erste Drehkupplungsteil,
- Fig. 6: eine Darstellung eines Stanzblechteils, das wesentliche Teile der Vorrichtung enthält, und
- Fig. 7: eine vergrößerte Schnittdarstellung einer Elektrode mit daran integriertem Wärmetauscher.

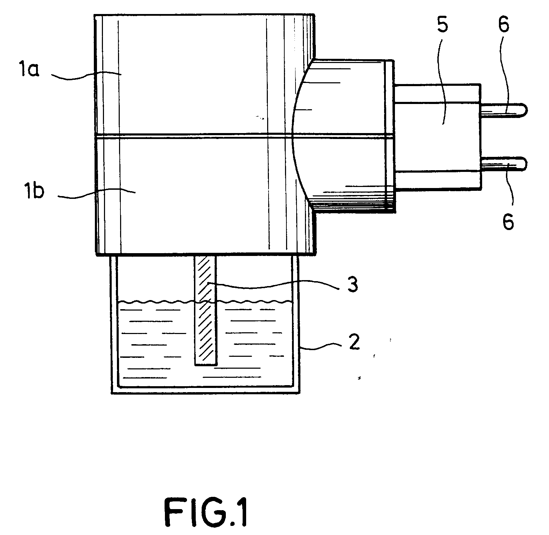

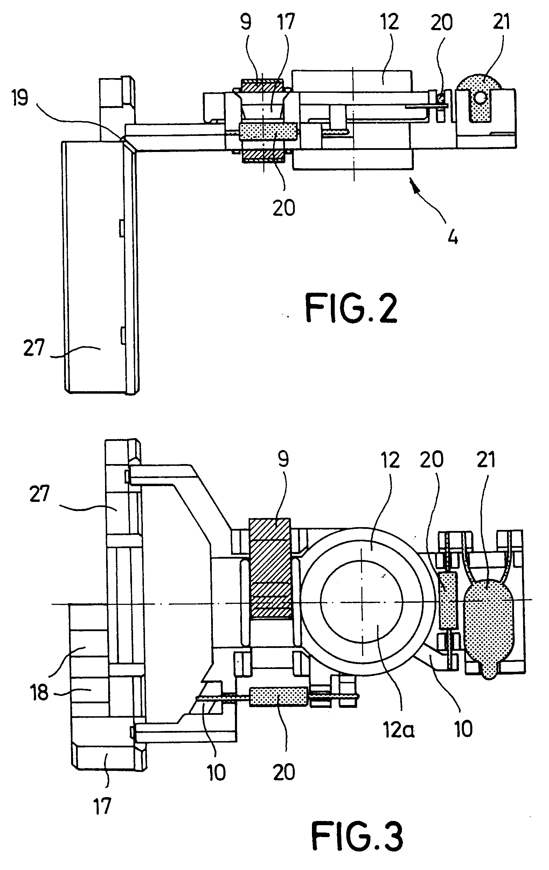

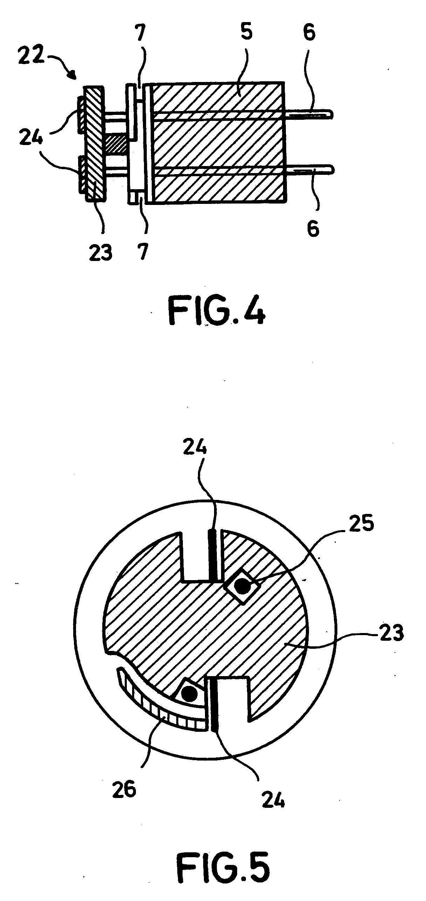

Gemäß Fig. 1 weist die Vorrichtung ein aus zwei Gehäusehalbschalen 1a, 1b bestehendes Gehäuse auf, mit einem daran befestigbaren Behälter 2, in der sich die zu verdunstende Substanz, beispielsweise eine Flüssigkeit mit darin gelösten Wirkstoffen befindet. Die Befestigungsmittel zwischen dem Gehäuse und dem Behälter 2 bestehen aus einem am oberen Ende des Behälters angeordneten Außengewinde und einem entsprechenden in der unteren Gehäusehalbschale 1b ausgebildeten Innengewinde. In dem Behälter 2 ist ein Docht 3 gelagert, der aus Kohlefasern oder Textilfäden besteht. Über den Docht wird die zu verdunstende Flüssigkeit zu der in den Fig. 2 und 3 näher dargestellten Heizung 4 transportiert. In dem Gehäuse ist ein Anschlußstecker 5 mit zwei daran befestigten Steckkontakten 6 drehbar gelagert. Wie aus Fig. 4 ersichtlich, weist der Anschlußstecker eine umlaufende Ringnut 7 auf, die bei geschlossenem Gehäuse in eine entsprechend ausgeformte Randkante der Gehäuseschalen 1a, 1b eingreift.

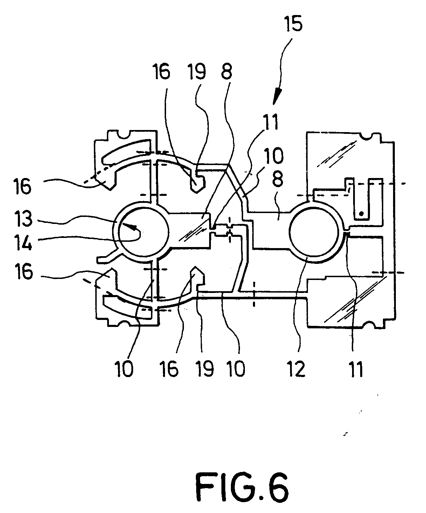

Die Heizung 4 umfaßt ein zwischen zwei übereinander angeordneten Elektroden 8 eingeklemmtes PTC-Heizelement, welches in den Fig. 2 und 3 nicht direkt sichtbar ist, da die Elektroden 8 größtenteils von einem wärmebeständigen und isolierenden Kunststoffmaterial umspritzt sind und eine freie Sicht auf das PTC-Element verhindern. Die Elektroden sind auf den voneinander abgewandten Seiten in dem Kunststoffmaterial eingebettet. Die einander zugewandten Seiten weisen eine Kontaktfläche auf, die groß genug ist, um das PTC-Element vollständig zu kontaktieren. Entlang des äußeren Randes der Elektroden ist ein umlaufender Steg ausgebildet, wobei die Stege auf der oberen und unteren Elektrode so angeordnet sind, daß sie ineinander eingreifen. Dadurch bildet sich ein abgeschlossener Hohlraum, in dem das PTC-Element formschlüssig gehalten ist. Zwischen der unteren Elektrode und dem PTC-Element befindet sich ein elastisches, elektrisch leitendes Element, welches zusammen mit einer von außen die Elektroden umfassenden U-förmigen Klemmfeder 9 für einen guten Kontakt zwischen den Elektroden und dem PTC-Element sorgt. Der elektrische Teil der Heizung umfaßt weiterhin Verbindungsleitungen 10, von denen ein Teil über einen Bereich 11 mit vermindertem Leitungsquerschnitt mit den Elektroden 8 verbunden sind, wie aus Fig. 6 deutlicher erkennbar ist.

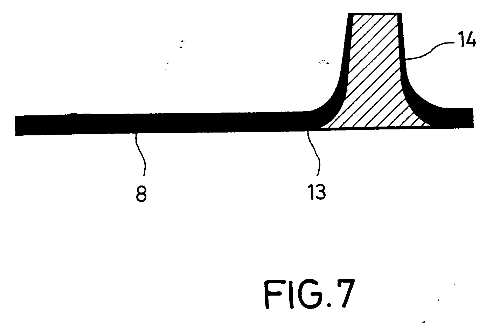

Ein Wärmetauscher 12 besteht aus zwei aufeinandergelegten ringförmigen Abschnitten 13, die einen tiefgezogenen Bereich 14 aus dem gleichen metallischen Material aufweisen, aus dem auch die Elektrode gebildet ist. Wie aus Fig. 7 deutlich wird, beträgt das Verhältnis der Länge des tiefgezogenen Bereichs 13 zur Dicke der Elektroden in etwa 6:1. Der Tiefziehvorgang wird so durchgeführt, daß sich die Materialstärke des tiefgezogenen Bereichs zu seinem freien Ende hin verjüngt. Insbesondere ist es bei Tiefziehen wichtig, daß ausreichend Material an der Ansatzstelle zwischen dem tiefgezogenen Bereich 14 und dem ringförmigen Abschnitt 13 vorhanden ist, damit ein guter Wärmeübergang auf dem tiefgezogenen Bereich gewährleistet ist. Die größere Materialstärke an den Stellen, die nahe am Heizelement liegen, führt dazu, daß die Heizenergie gut bis an die äußeren Enden übertragen wird. Der ringförmige und tiefgezogene Bereich 13, bzw. 14 sind vollständig in dem isolierten Kunststoffmaterial eingebettet.

In Fig. 3 erkennt man die umspritzten, ringförmigen und tiefgezogenen Bereiche des Wärmetauschers 12, der einen axial zentrierten Durchgangskanal 12a aufweist. Der ringförmige Bereich ist an seiner Innenwand nur mit einer dünnen Isolierschicht überzogen, damit möglichst viel Wärmeenergie in den Durchgangskanal 12a abgeführt werden kann. Dagegen besitzt die Außenwand des ringförmigen Bereichs eine dicke Isolierschicht, die sich in axialer Richtung des tiefgezogenen Bereichs zu seinem freien Ende hin verstärkt. Eine so ausgebildete Isolation vermindert die Wärmeverluste und sorgt dafür, daß die zu übertragende Wärme genau an die gewünschte Stelle im Durchgangskanal transportiert wird.

Im folgenden sollen die wesentlichen Schritte zur Herstellung einer Vorrichtung nach der vorliegenden Erfindung dargestellt werden.

Fig. 6 zeigt ein Stanzblechteil 15 mit den wesentlichen Teilen der elektrischen Heizung. Im vollständig ausgestanzten Zustand des Blechteils sind die Stellen, die in der Zeichnung mit gestrichelten Linien gekennzeichnet sind, vollständig durchtrennt. Im einzelnen erkennt man zwei Elektroden 8 mit daran integral ausgeformten ringförmigen Abschnitten 13, die jeweils einen tiefgezogenen Bereich 14 aufweisen. Integral mit den Elektroden 8 bzw. dem ringförmigen Abschnitt 13 sind Verbindungsleiter 10 verbunden, zum Teil über Bereiche 11 mit vermindertem Querschnitt. Die Verbindungsleiter sind in der linken Hälfte des in Fig. 6 gezeigten Stanzteils etwa viertelkreisförmig und einander zugewandt ausgebildet und tragen jeweils ein Paar jeweils um 90° versetzte Kontakte 16.

In einem weiteren Verfahrensschritt wird das Stanzblechteil an den Stellen, an denen eine erhöhte Formsteifigkeit bzw. eine Wärmeisolierung erforderlich ist, mit einem Kunststoff umspritzt. Die Umspritzung der Viertelkreisringe wird so vorgenommen, daß sich ein nahezu geschlossener Kreisring ausbildet, an dessen Innenwand die Kontakte 16 radial nach innen ragen. An der Stirnseite des Kreisrings ist ein Steg 17 ausgebildet, der sich in axialer Richtung des Kreisrings erstreckt. Der Steg verläuft auf einer Länge von ca. einem Viertel des Kreisrings und weist in seiner Innenwandung eine Vielzahl von Vertiefungen 18 mit abgeschrägten Flächen auf (Fig. 3). Nach dem Umspritzen werden die in Fig. 6 mit gestrichelten Linien gezeichneten Stellen durchtrennt, wodurch die in der linken Hälfte gezeigte Elektrode vollständig herausfällt. Danach werden die in dem nahezu geschlossenen Kreisring eingebetteten viertelkreisringförmigen Verbindungsleiter an den Stellen 19 um etwa 90° gegenüber dem verbleibenden elektrischen Teil der Heizung abgebogen, was aus Fig. 2 ersichtlich ist.

Schließlich wird die Heizung mit Widerständen 20 und einer Leuchtdiode 21 vollständig bestückt.

Im Betrieb der Vorrichtung funktioniert der elektrische Schaltkreis der Heizung wie folgt: Ausgehend von einer Verbindungsleitung 10 fließt der Heizstrom über einen Sicherungswiderstand 20 zum oberen kreisringförmigen Abschnitt 13, der integral mit der oberen Elektrode 8 ausgebildet ist. Von dieser Elektrode fließt der Strom durch das PTC-Heizelement hindurch zur unteren Elektrode 8 und von dort zu einer zweiten Verbindungsleitung 10, welche vollständig vom Kunststoffmaterial umspritzt ist. Die beiden Verbindungsleitungen 10 sind mit den in Fig. 1 gezeigten Steckkontakten 6 des Anschlußsteckers 5 verbunden. Parallel zu dem Elektrodenpaar 8 ist ein Vorwiderstand 20 mit einer dazu in Reihe geschalteten Leuchtelektrode 21 geschaltet. Die Verbindungsleitungen für den Vorwiderstand und die Leuchtelektrode befinden sich jeweils an dem oberen und unteren kreisringförmigen Abschnitt 13 des Wärmetauschers 12.

PCT-Heizelemente sind dafür bekannt, daß sie sich bei einer von ihrer Dimensionierung abhängigen Temperatur selbst stabilisieren. Für das vorliegende Ausführungsbeispiel wurde das PTC-Heizelement so ausgewählt, daß es bei der vorgegebenen Betriebsspannung eine Temperatur von 150°C erzeugt. Da sich die obere und untere Elektrode 8 in innigem Kontakt mit dem Heizelement befindet und die ringförmigen Abschnitte mit dem tiefgezogenen Bereich einstückig daran ausgebildet sind, wird die vom Heizelement erzeugte Wärme zum Wärmetauscher 12 transportiert.

Aufgrund der erfindungsgemäßen Ausgestaltung der Vorrichtung sind die Wärmeverluste so gering, daß in dem Durchgangskanal am-Docht eine Temperatur von ca. 128° gemessen wurde. Bemerkenswert an diesem Ergebnis ist der geringe Temperaturverlust von nur 22°C gegenüber der Betriebstemperatur des PTC. Weiterhin ist bei Dauerversuchen eine sehr geringe Temperaturschwankung von nur 0,5°C nachgewiesen worden.

Es soll darauf hingewiesen werden, daß die dargestellte Ausführung der Heizung nur eine beispielhafte Möglichkeit darstellt und das vielmehr mehrere technische Realisierungsmöglichkeiten der die Heizung existieren. Zum Beispiel muß das Heizelementder nicht unbedingt aus einem PTC-Heizelement bestehen, sondern kann auch eine Heizwicklung aus einem Heizdraht sein, die in einem keramischen Heizkörper eingebettet ist.

Eine andere, in den Zeichnungen nicht dargestellte Ausführungsform der vorliegenden Erfindung, unterscheidet sich von der oben beschriebenen Ausführung durch eine andere Ausbildung des Wärmetauschers 8. Bei dieser Ausführungsform werden die kreisringförmigen Abschnitte 13 und der tiefgezogene Bereich 14 durch eine ebene Heizfläche ersetzt. Weiterhin wird anstelle des Behälters 2 mit dem darin gehaltenen Docht ein Behälter vorgesehen, der sich oberhalb der Heizfläche befindet, beispielsweiser eine wärmebeständige Plastikschale. In der Schale befindet sich die zu verdunstende Substanz, welche direkt von der darunter angeordneten Heizfläche gleichmäßig beheizt wird. Bei diesem Ausführungsbeispiel weist das Gehäuse eine geeignete Öffnung auf, durch die der Behälter mit der zu verdunstenden Substanz eingebracht werden kann.

Im folgenden soll Bezug genommen werden auf die Fig. 4 und 5, welche die Ausbildung eines ersten Drehkupplungsteils 22 erläutern.

Das erste Drehkupplungsteil 22 ist starr mit dem Anschlußstecker 5 verbunden und besteht im wesentlichen aus einem scheibenförmigen Trägerteil 23, welches zwei sich gegenüberstehende streifenförmige elektrische Kontakte 24 trägt. Die elektrischen Kontakte 24 sind über Kontaktstifte 25 (Fig. 5) mit den Steckkontakten 6 verbunden. Am äußeren Umfang des scheibenförmigen Trägerteils 23 ist ein Nocken 26 ausgebildet, der an seinem freien Ende zwei Flächen aufweist, die einen Winkel von etwa 120° miteinander einschließen.

Ein zweites Drehkupplungsteil 27 besteht im wesentlichen aus dem nahezu geschlossenen Kreisring, der aus der Umspritzung der viertelkreisringförmig ausgebildeten Verbindungsleitungen 10 entstanden ist (vgl. Fig. 2 und 3) und aus dem damit verbundenen Steg 17, der eine Anzahl Vertiefungen aufweist.

Wenn die erfindungsgemäße Vorrichtung fertig montiert ist, greift das Trägerteil 23 des ersten Drehkupplungsteils 22 in das kreisringförmige zweite Drehkupplungsteil 27 ein. Der an dem Trägerteil 23 ausgebildete Nocken 26 greift dabei in die Vertiefungen 18 ein, die an dern Innenwandung des zweiten Drehkupplungsteils ausgebildet sind. Wenn der Anschlußstecker 5 mit dem daran befestigten ersten Drehkupplungsteil gegenüber dem Gehäuse gedreht wird, gleitet der Nocken 26 rastend über die Vertiefungen 18 des zweiten Drehkupplungsteils. Die Verdrehung des Anschlußsteckers 5 gegenüber dem Gehäuse ist mittels einer nicht näher gezeigten Begrenzungseinrichtung auf etwa 90° begrenzt.

## Patentansprüche

1. Vorrichtung zum Verdunsten von Wirkstoffen, Parfümen oder dergleichen flüchtigen Substanzen, mit einem Gehäuse, in dem eine elektrische Heizung angeordnet ist und in das ein, den Wirkstoff enthaltender Behälter einsetzbar ist, einem Wärmetauscher, der mit der Heizung in Wärmekontakt steht und an einer Stelle angeordnet ist, an der der Wirkstoff zum Verdunsten gebracht werden soll, ferner mit einem, mit dem Gehäuse verbundenen Anschlußstecker für eine Wandsteckdose,
**dadurch gekennzeichnet,**
daß der Anschlußstecker (5) mit einem ersten Drehkupplungsteil (22) versehen ist, das mit einem im Gehäuse (1a, 1b) angeordneten zweiten Drehkupplungsteil (27) in Eingriff steht und daß in jeden der Drehkupplungsteile (22, 27) Kontakte (16) angeordnet sind, die in und außer Kontakt mit den ihnen zugeordneten Gegenkontakten (16) des jeweils anderen Drehkupplungsteils (27, 22) bringbar sind, wobei die Kontakte und die zugehörigen Gegenkontakte auf den beiden Drehkupplungsteilen derart angeordnet sind, daß sich in zwei um 90° versetzten Stellungen jeweils eine durchgehende elektrische Verbindung zwischen den Anschlußsteckerkontakten (6) und der Heizung (4) ergibt und dazwischen ein Winkelbereich mit einer Schaltstellung entsteht, in der die Verbindung geöffnet ist.

2. Vorrichtung nach Anspruch 1, **dadurch gekennzeichnet,** daß der Winkelbereich mit geschlossener Verbindung wesentlich kleiner ist, als der Winkelbereich mit geöffneter Verbindung, vorzugsweise mit einem Faktor kleiner als 1:5.

3. Vorrichtung nach Anspruch 1 oder 2, **dadurch gekennzeichnet,** daß die Heizung (4) wenigstens einen PTC-Widerstand aufweist, der zwischen zwei metallischen Elektroden (8) liegt, die über elektrische Verbindungsleitungen (10) mit den Kontakten (16) des gehäuseseitigen Drehkupplungsteils (27) verbunden sind.

4. Vorrichtung nach Anspruch 3, **dadurch gekennzeichnet,** daß die Verbindungsleitungen (10), die Kontakte (16), der Wärmetauscher (12) und gegebenenfalls die Elektroden (8) einstückig aus einem Stanzblechteil (15) gebildet sind, das nach dem Stanzen erforderlichenfalls abgebogen und an den Stellen, an denen eine erhöhte Formsteifigkeit bzw. eine Wärmeisolierung erforderlich ist, mit einem Kunststoff umspritzt ist.

5. Vorrichtung nach Anspruch 4, **dadurch gekennzeichnet,** daß die elektrischen Verbindungsleitungen (10) in dem Bereich zwischen den Kontakten (16) des gehäuseseitigen Drehkupplungsteils (27) in Form zweier einander gegenüberliegender Viertelringe ausgebildet sind, daß die Verbindungsleitungen (10) an einer Stelle (19) zwischen den Viertelringen und den Elektroden (8) um einen Winkel von etwa 90° gegenüber den Elektroden (8) abgeknickt sind und daß die Viertelringe mit Kunststoff derart umspritzt sind, daß sich ein nahezu geschlossener Kreisring ergibt.

6. Vorrichtung nach einem der Ansprüche 1 bis 5, **dadurch gekennzeichnet,** daß das erste Drehkupplungsteil eine starre Verbindung mit dem Anschlußstecker (5) aufweist und in der Wand des Gehäuses (1a, 1b) vorzugsweise um einen Winkel von 90° drehbar geführt ist.

7. Vorrichtung nach wenigstens einem der Ansprüche 1 bis 6, **dadurch gekennzeichnet,** daß das erste Drehkupplungsteil (22) mittels einer Rastverbindung (18, 26) mit dem zweiten Drehkupplungsteil (27) in Eingriff gehalten ist.

## Claims

1. A device for the vaporization of active substances, perfumes or similar volatile substances, comprising a housing, in which an electric heating is disposed and into which a container containing the active substance can be inserted, a heat exchanger, which is in heating contact with the heating and which is disposed at a position at which the active substance shall be evaporated, further comprising a plug for a wall socket connected to the housing,
**characterized in** that
the plug (5) is provided with a first rotating joint member (22), engaging a second rotating joint member (27) disposed within the housing (1a, 1b) and that contacts (16) are arranged in each of the rotating joint members (22, 27), which can be brought into and out of contact with counter-contacts (16) of the respective other rotating joint member (27, 22) associated thereto, wherein the contacts and the respective counter-contacts are arranged on the two rotating joint members in a manner that a continuous electrical connection between the plug contacts (6) and the heating (4) results in two positions offset about 90°, and that an angular range with a switch position results thereinbetween in which the connection is opened.

2. A device according to claim 1, **characterized in** that the angular range with a closed connection is considerably smaller than the angular range with an opened connection, preferrably at a factor which is smaller than 1:5.

3. A device according to claim 1 or 2, **characterized in** that the heating (4) comprises at least one PTC resistor, which is disposed between two metallic electrodes (8), which are connected through electric connecting lines (10) to the contacts (16) of the rotating joint member (27) near the housing.

4. A device according to claim 3, **characterized in** that the connecting conduits (10), the contacts (16), the heat exchanger (12) and possibly the electrodes (8) are integrally formed of a punching sheet (15), which is bent after punching, if necessary, and which is coated by a plastic material at the points at which an increased inherent stability or heat insulation is required.

5. A device according to claim 4, **characterized in** that the electric connecting conduits (10) are provided between the contacts (16) of the rotating joint member (27) close to the housing in the form of two opposing quarters of a ring, and that the connecting conduits (10) are bent about an angle of approximately 90° with respect to the electrodes (8) at a position (19) between the quarters of a ring and the electrodes (8), and that the quarters of a ring are coated by plastic material in a manner that an almost closed ring results.

6. A device according to one of claims 1 to 5, **characterized in** that the first rotating joint member comprises a rigid connection with the plug (5) and is rotatably guided in the wall of the housing (1a, 1b) preferrably about an angle of 90°.

7. A device according to at least one of claims 1 to 6, **characterized in** that the first rotating joint member (22) engages the second rotating joint member (27) through a lock-in connection (18, 26).

## Revendications

1. Dispositif de vaporisation de substances actives, de parfums ou de substances volatiles similaires, comprenant un boîtier dans lequel est disposé un chauffage électrique et dans lequel peut être placé un récipient contenant la substance active, un échangeur thermique qui est en contact thermique avec le chauffage et est disposé à un endroit sur lequel la substance active doit être apportée aux fins de vaporisation, comprenant en outre une fiche de raccordement reliée au boîtier pour une prise murale, caractérisé en ce que la fiche de raccordement (5) est dotée d'une première partie à joint tournant (22), qui est en prise avec une deuxième partie à joint tournant (27) disposée dans le boîtier (1a, 1b) et en ce que dans chacune des parties à joint tournant (22, 27) sont disposés des contacts (16) qui peuvent être mis à chaque fois en et hors contact avec les contre-contacts leur étant associés (16) de l'autre partie à joint tournant (27, 22), les contacts et les contre-contacts allant avec étant disposés sur les deux parties à joint tournant de manière telle qu'il en résulte à chaque fois, dans deux positions décalées à 90°, une connexion électrique continue entre les contacts de la fiche de raccordement (6) et le chauffage (4) et qu'entre les deux se forme une zone angulaire avec une position de commutation, dans laquelle la connexion est ouverte.

2. Dispositif selon la revendication 1, caractérisé en ce que la zone angulaire avec la connexion fermée est essentiellement plus petite que la zone angulaire avec la connexion ouverte, de préférence avec un facteur plus petit que 1:5.

3. Dispositif selon la revendication 1 ou 2, caractérisé en ce que le chauffage (4) présente au moins une résistance à coefficient positif de température, qui se trouve entre deux électrodes métalliques (8) qui sont reliées par des lignes de connexion électriques (10) aux contacts (16) de la partie à joint tournant (27) du côté du boîtier.

4. Dispositif selon la revendication 3, caractérisé en ce que les lignes de connexion (10), les contacts (16), l'échangeur thermique (12) et, le cas échéant, les électrodes (8) sont formés d'une seule pièce dans une partie de tôle à estamper (15), qui est pliée si nécessaire après l'estampage et est couverte par extrusion d'une matière synthétique aux endroits sur lesquels une résistance propre accrue ou bien une isolation thermique est nécessaire.

5. Dispositif selon la revendication 4, caractérisé en ce que les lignes de connexion (10) sont constituées, dans la zone entre les contacts (16) de la partie à joint tournant (27) du côté du boîtier, sous la forme de deux quarts d'anneau opposés l'un à l'autre, en ce que les lignes de connexion (10) sont coudées à 90° environ par rapport aux électrodes (8) à un endroit (19) entre les quarts d'anneau et les électrodes (8) et en ce que les quarts d'anneau sont couverts de matière synthétique par extrusion de telle manière qu'il en en résulte un anneau presque fermé.

6. Dispositif selon l'une des revendications 1 à 5, caractérisé en ce que la première partie à joint tournant présente une liaison rigide avec la fiche de raccordement (5) et peut être guidée de préférence en pouvant tourner selon un angle de 90° dans la paroi du boîtier (1a, 1b).

7. Dispositif selon l'une au moins des revendications 1 à 6, caractérisé en ce que la première partie à joint tournant (22) est maintenue en prise avec la deuxième partie à joint tournant (27) au moyen d'une jonction à crans d'arrêt (18, 26).
